# FASCICULE DE BREVET EUROPEEN

(11) **EP 0 516 508 B1**
(45) Date de publication et mention de la délivrance du brevet: **19.07.1995**
(21) Numéro de dépôt: 92401376.6
(22) Date de dépôt: 20.05.1992
(51) Int. Cl.: A61K 7/00, A61K 7/46

(54) **Microémulsion contenant un concentré parfumant et produit corresponant**
Parfümierendes Konzentrat enthaltende Mikroemulsion und entsprechendes Produkt
Microemulsion containing a perfuming concentrate and corresponding product

(30) Priorité: 31.05.1991 FR 9106592
(43) Date de publication de la demande: 02.12.1992
(73) Titulaire: YVES SAINT LAURENT PARFUMS, (Société Anonyme), 92200 Neuilly sur Seine (FR)
(72) Inventeur: Dartnell, Nathalie, F-75017 Paris (FR); Breda, Bernard, 78380 Bougival (FR)
(74) Mandataire: Lerner, François

(56) Documents cités:
- EP-A- 0 278 660
- EP-A- 0 334 777
- EP-A- 0 368 146
- GB-A- 2 190 681
- SCHICK ET AL. 'SURFACTANT SCIENCES SERIES, VOL. 6, EMULSIONS
- & EMULSION TECHNOLOGY, PT. II.' , DEKKER INC. , NEW YORKUSA

## Description

L'invention se rapporte d'une façon générale à un composé parfumant à usage cosmétique (incluant l'usage pharmaceutique) pouvant avoir une relativement forte concentration en concentré de parfum, avec un aspect transparent à l'image des produits parfumants classiques de nature alcoolique.

Comme on le sait, les parfums "cosmétiques" sont aujourd'hui des produits vendus sous la dénomination "parfum" ou "extrait" et renferment en une concentration relativement importante, habituellement de l'ordre de 15 à 30 %, un mélange de produits odorants, naturels ou synthétiques, en solution dans un alcool, tel que de l'alcool éthylique, et quelquefois d'autres solvants, avec ou sans addition d'eau ou d'autres adjuvants.

Les autres produits parfumants actuellement connus, tels que les "parfums de toilette" ou "eaux de parfum", et les "eaux de toilette" présentent globalement d'une façon générale la même composition parfumée mais contiennent de plus faibles concentrations de matières odorantes, habituellement en solution dans un alcool moins concentré. D'une façon générale, on considère que les parfums de toilette ou eaux de parfum présentent une concentration en produits de parfum comprise entre environ 8 et 15 %, tandis que les eaux de toilette présentent une concentration généralement de l'ordre de 4 à 8 % (en poids du produit parfumant).

Il s'avère toutefois que la présence de concentrations toujours relativement élevées d'alcool dans les produits parfumants actuels limite considérablement leur utilisation.

En effet, l'alcool est connu comme étant un irritant cutané en ce sens qu'il déssèche la peau par élimination du film hydrolipidique recouvrant et protégeant la surface cutanée. Il constitue de plus un irritant oculaire primaire et tend à favoriser au niveau capillaire la formation de pellicules, en ternissant de surcroît les cheveux et en les rendant secs et cassants par désorganisation de la cuticule et par élimination de l'enduit sébacé protecteur.

Néanmoins, comme on le sait, l'alcool est classiquement considéré comme étant pratiquement le seul composé chimique permettant la solubilisation de concentrés de parfums, de nature exclusivement lipophile, en une seule phase homogène et transparente, ces concentrés de parfums (ou de matières odorantes) étant souvent obtenus par extraction par les solvants ou par distillation de substances d'origine végétale ou animale, voire à partir de produits de synthèse.

Or, l'invention a justement pour objet un produit parfumant non alcoolique présentant, de façon avantageuse, un caractère transparent et stable, une excellente innocuité, une très bonne tolérance cutanée, une quasi absence de toxicité et pratiquement aucune action détergente.

Dans le cadre de l'invention, on considèrera que le produit parfumant en cause est non alcoolique dans la mesure où il ne contient aucun hydrocarbure saturé avec un nombre d'atomes de carbone inférieur à 10 ou supérieur à 20, porteur d'une fonction hydroxyle en substitution d'une fonction hydrogène (l'éthanol étant l'exemple typîque d'un tel alcool).

Le produit en question devant éventuellement pouvoir être diffusé dans les cheveux, à titre de produit parfumant non irritant, ni dégradant, l'utilisation de composés alcooliques est de surcroît apparue comme devant réellement être évitée.

C'est pourquoi, l'invention propose plus précisément un produit cosmétique parfumant non alcoolique comprenant une phase hydrophile et une phase lipophile renfermant un concentré de parfum(s) ou matières odorantes, ces phases étant associées dans une microémulsion et la teneur en matières odorantes étant comprise entre environ 5 et 50 % en poids du produit, de préférence.

On notera dès à présent qu'il y a lieu de distinguer,dans le domaine cosmétique, les microémulsions à usage (comme en l'espèce) de produits parfumants, des microémulsions plus classiques "parfumées" dans lesquelles le concentré de parfum n'est ajouté que dans de faibles concentrations (par exemple de l'ordre de 1 à 3 % environ) dans le but essentiel de couvrir l'odeur résiduelle liée aux matières premières constitutives de la microémulsion.

Et cette distinction est d'autant plus à faire dans la mesure où il est apparu qu'augmenter jusqu'à des doses pouvant être très importantes la concentration de parfum dans une microémulsion entraîne des problèmes accrus de solubilité du concentré de parfum dans cette microémulsion et de stabilité de cette dernière, ainsi que des problèmes d'oxydation favorisés par la phase hydrosoluble souvent constituée d'eau, problèmes qui ne se rencontraient pas bien entendu dans le cadre des produits alcooliques.

En association avec le produit parfumant précité, l'invention vise donc tout naturellement la microémulsion qui renferme le concentré parfumant.

En l'espèce cette microémulsion se caractérise en ce qu'elle comprend, en mélange :
- une phase aqueuse ou hydrophile,
- une phase grasse de nature lipophile comprenant un concentré parfumant constitué de matières odorantes,
- au moins un agent tensio-actif ou émulsifiant, à base de polyéthylène glycol,
- un premier agent cotensio-actif (ou co-émulsifiant) à base de polyglycérol,
- et un deuxième agent cotensio-actif (ou co-émulsifiant) à base d'éther phosphate.

Il s'agit bien entendu là d'une microémulsion consistant en une solution micellaire homogène et quasi transparente, renfermant des particules ayant des dimensions comprises entre environ 5 et 500 angströms (10⁻¹⁰m).

Il est également clair que tant l'agent tensioactif que les deux agents cotensio-actifs sont ici employés essentiellement en tant qu'agents d'émulsion, voire en tant que stabilisateurs d'émulsion ou agents d'augmentation de la viscosité. Une liste de tels agents est fournie dans le manuel des ingrédients cosmétiques CTFA (pages 90 à 94).

Si l'on se reporte notamment à la demande de brevet européen EP-A-0 334 777 on pourra constater qu'il était déjà connu d'utiliser notamment dans l'industrie cosmétique une microémulsion comprenant, comme en l'espèce, une phase hydrophile (telle que de l'eau, des glycols, du glycérol, voire des mélanges de ces composés), un agent tensio-actif à base de polyéthylène glycol et un agent cotensio-actif à base de polyglycérol.

Toutefois, on constatera que dans ce document la phase hydrophile était associée nécessairement à une phase huileuse de nature lipidique, telle qu'une huile minérale, végétale, animale ou synthétique.

Or, dans le cadre de l'invention, une telle partie lipidique n'est pas apparue comme indispensable, ayant pu être remplacée par une phase grasse de nature lipophile, renfermant le concentré de parfum retenu, et pouvant être globalement non lipidique.

Pour éviter tout malentendu, on définira comme lipophile un groupement moléculaire globalement apolaire au niveau duquel s'exercent particulièrement les attractions ou affinités à l'égard des molécules d'un milieu organique à caractère hydrophobe prédominant, et notamment des lipides, ces lipides étant définis comme des substances lipophiles insolubles dans l'eau, solubles dans le benzène et l'éther et renfermant un ou plusieurs acides gras ou dérivés d'acides gras tels que esters, alcools et aldéhydes.

Dans la publication EP-A-0 334 777, il est également indiqué que si les "agents tensio-actifs" sont des composés chimiques à caractère hydrophile prononcé, ils ne sont destinés qu'à provoquer la formation de micelles, un agent tensio-actif utilisé seul permettant uniquement de former ainsi une solution micellaire, tandis que les agents cotensio-actifs ont un caractère plus hydrophobe et sont destinés plus particulièrement à provoquer la solubilisation mutuelle des phases de la microémulsion.

Toujours dans ce document, il est en outre indiqué que l'association d'un agent tensio-actif à base de polyéthylène glycol et d'un agent contensio-actif à base de polyglycérol permet d'obtenir une microémulsion stable thermiquement et mécaniquement.

Toutefois, rien n'indique qu'une telle stabilité pouvait également être atteinte en présence d'une phase lipophile avantageusement non lipidique essentiellement à base, de surcroît, d'un concentré parfumant pouvant atteindre 50 % environ en poids du produit ou de la microémulsion.

Or, il s'est avéré que même essentiellement en l'absence d'alcool (voir définition préalablement donnée), un tel composé demeurait stable à la fois thermiquement et mécaniquement.

Il est également apparu que dans la microémulsion de l'invention la présence d'un deuxième agent cotensio-actif à base d'éther phosphate favorisait encore la solubilisation des composés et la stabilité d'ensemble de la microémulsion.

De surcroît il a été noté une nette diminution des problèmes d'oxydation, problèmes en l'espèce accrus compte tenu de la phase hydrophile qui peut être très essentiellement à base d'eau ou de composés aqueux.

Une explication avancée est que ce second agent cotensio-actif (ou co-émulsionnant), dans la mesure où il permet d'accroître la stabilité notamment dans les fortes teneurs en composé parfumant, permettrait aux micelles de cette microémulsion de mieux "enrober" le concentré en cause et donc de le protéger à la fois contre les rayonnements extérieurs (notamment ultraviolets) et contre le milieu hydrophile, en particulier s'il est aqueux.

La microémulsion ainsi obtenue présente donc une excellente innocuité, une très bonne tolérance cutanée et une quasi absence de toxicité sans pratiquement d'action détergente.

Pour la définition de toutes ces expressions on se reportera avantageusement si nécessaire à la publication précitée EP-A-0 334 777.

En ce qui concerne l'agent tensio-actif ou émulsionnant utilisé, on notera encore qu'il s'agit avantageusement d'un ester mixte de glycérol et de polyéthylène glycol, présent à hauteur d'au moins environ 10 % en poids du mélange, cette frontière étant dictée dans la pratique par le fait qu'en dessous de cette teneur on tend à obtenir non plus une microémulsion mais simplement une émulsion traditionnelle, c'est-à-dire opaque . Quant à la la teneur maximale, elle sera essentiellement dictée par la concentration en concentré de matières odorantes, pouvant ainsi atteindre 50 % (voire plus) en poids du mélange. Dans cet agent tensio-actif, le polyéthylène glycol aura avantageusement un poids moléculaire moyen compris entre 100 et 1000, et de préférence de l'ordre de 400, lui conférant un caractère parfaitement non détergent.

Concernant le premier agent cotensio-actif d'émulsion, on choisira de préférence un ester de polyglycérol et d'acides gras liquides avec une proportion dans le mélange inférieure à celle de l'agent tensio-actif précité.

Avantageusement, dans ce premier agent cotensio-actif, le polyglycérol aura un poids moléculaire compris entre environ 100 et 1000 et l'acide gras pourra par exemple être constitué par l'acide oléique ou l'acide isostéarique, seuls ou en mélange.

En tant que teneur, celle du premier cotensioactif sera de préférence comprise entre environ 1 et 20 % en poids du mélange.

A titre de deuxième agent cotensio-actif, l'utilisation d'un alkyl éther phosphate basé sur des alcools gras polyéthoxylés associés dans le mélange en une proportion de préférence inférieure à celle de l'agent tensio-actif est apparue comme pouvant être conseillée.

En tant que concentration, on pourra en particulier prévoir une teneur en second agent cotensioactif comprise entre environ 0,5 et 15 % en poids du mélange.

En ce qui concerne le concentré de parfum utilisé en phase lipophile, sa concentration variera en pratique entre environ 8 et 20 % en poids si l'on veut faire entrer le produit dans la catégorie "parfum de toilette" ou "eau de parfum", et entre environ 15 et 30 % en poids si l'on veut faire entrer ce même produit dans la catégorie des "parfums" ou "extraits de parfum".

Parmi les composants des différents types de parfums qui pourront être utilisés, on peut notamment citer : les mélanges complexes de composés organiques multiples comme des huiles essentielles odorantes, esters, éthers, aldéhydes, certains alcools et hydrocarbures (hors les hydrocarbures saturés à nombre d'atomes de carbone inférieur à 10 ou supérieur à 20), les cétones, lactones, mais également d'autres classes de composants tels que les pyrrones et pyrroles. Pour une liste encore plus détaillée, on pourra se reporter à la publication EP-A-0368 146 (priorité US-88/267 872) dont le contenu, en ce qui concerne les composants parfumants utilisables, est inclus à la présente description, par référence.

A titre de constituant complémentaire,la microémulsion pourra de surcroît intégrer dans sa phase grasse une partie lipidique, pouvant être alors constituée par tout composé huileux, liquide d'usage courant pour les applications pharmaceutiques et/ou cosmétiques (tel que huile minérale, animale, végétale, siliconée, à base d'éthers d'acides gras organiques...), l'emploi de composés lipidiques ayant une balance hydrophile/lipophile (HLB) voisine de 2 pouvant s'avérer avantageux.

Bien entendu, la distinction entre les parties lipidique et lipophile sera d'autant plus marquée que la teneur en concentré de parfum sera plus forte et celle en "huile" plus faible, cette huile pouvant permettre de faire varier le HLB et de favoriser un aspect soyeux une fois le produit déposé par exemple sur les cheveux.

Concernant maintenant la phase hydrophile ou aqueuse, sa composition pourra renfermer, outre de l'eau ou un composé aqueux, des agents conservateurs, agents humectants et des principes actifs, lesquels principes actifs pourraient également, pour certains, appartenir à la phase grasse.

Comme cela est bien connu, les agents conservateurs ou antiseptiques sont destinés à protéger une composition (en l'espèce la microémulsion)contre en particulier les attaques microbiennes. Il peut s'agir notamment de parabens, comme du méthyl-para-hydroxy benzoate (methyl para ben), du propyl-para-hydroxy benzoate, de l'imidazolidinyl urée... Pour toute description plus détaillée, on se reportera au brevet US-A-4 917 891 dont le contenu (en ce qui concerne les agents conservateurs) est inclus par référence.

En tant qu'agent humectants destinés à retenir l'humidité en surface de la peau, on peut notamment citer les polyols, ou les alcools polyhydriques tels que glycérine, sorbitol, propylène glycol...

Quant aux principes actifs, on les définira comme constitués à la base de toute matière première d'origine végétale animale ou synthétique entrant dans la composition d'un cosmétique, habituellement autre que les excipients, et ayant des propriétés biologiques favorables à l'épiderme humain, telles que des propriétés d'hydratation des couches superficielles de l'épiderme, de régénération cellulaire, ou encore des propriétés adoucissantes, émollientes, nettoyantes, apaisantes,... Cela concerne donc aussi bien des vitamines queu les acides gras essentiels, les huiles végétales (par exemple macadamia, amande douce, bourrache,...), les huiles animales (par exemple sqalane), les enzymes (SOD), les glycosaminoglycanes (tels que l'acide hyaluronique), les protéines (telles que le collagène, l'élastine,...) plus ou moins purifiées.

En pratique, les principes actifs sont ainsi souvent utilisés pour conférer à la peau des propriétés d'hydratation (glycosaminoglycanes, acides gras essentiels,...), de souplesse et d'élasticité (huile végétale, protéine,...) ou de protection contre un disfonctionnement métabolique ou encore contre les effets néfastes des rayonnements ultraviolets.

Le mélange de ces différents composés s'effectuera d'une façon générale dans des proportions préalablement déterminées par l'expérience, sans précaution particulière ou ordre spécifique, notamment à température ambiante et sous faible agitation, la microémulsion pouvant, en fonction des besoins, être de nature huile dans eau ou eau dans huile..

Les exemples qui suivent donnés à titre non limitatifs permettent d'apprécier quelques conditions d'essais concluants.

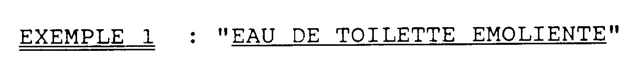

Dans un becher, on mélange à température ambiante :
- 10 à 15 g de tensio-actif glycérides C8-C10 polyglycolysés,
- 1 à 3 g de cotensio-actif dioléate de polyglycérol,
- 0,5 à 2 g de cotensio-actif DEA alcool oléique polyéthoxylé éther phosphate,
- 10 g d'huile végétale,
- 4 à 5 g de concentré de parfum,
- 0,2 g de conservateur,
- qsp 100 g d'eau déminéralisée.

Après environ 15 minutes d'agitation à vitesse lente à température ambiante (20 à 25°C), on obtient une microémulsion fluide et transparente.

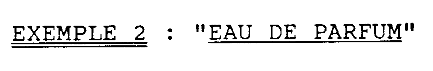

Dans un becher, on mélange à température ambiante :
- 20 à 26 g de tensio-actif glycéryl stéarate polyglycolysé,
- 2 à 6 g de cotensio-actif dioléate de polyglycérol,
- 1 à 6 g de cotensio-actif DEA alcool oléique polyéthoxylé éther phosphate,
- 15 g de concentré de parfum,
- 4 g d'huile végétale,
- 1 g d'extrait protéique,
- 0,2 g de conservateur,
- qsp 100 g d'eau déminéralisée.

Après environ 15 minutes d'agitation à vitesse lente à température ambiante, on obtient une microémulsion fluide et transparente.

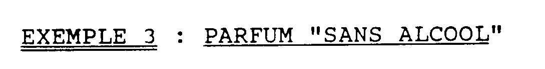

Dans un becher, on mélange à température ambiante :
- 30 à 50 g de tensio-actif glycéryl stéarate polyglycolysé,
- 10 à 15 g de cotensio-actif isostéarate de polyglycérol,
- 4 à 10 g de cotensio-actif sodium dioléate polyéthoxylé éther phosphate,
- 30 à 50 g de concentré de parfum,
- 0,2 g de conservateur,
- qsp 100 g d'eau déminéralisée.

Après environ 30 minutes d'agitation modérée à température ambiante, on obtient une microémulsion transparente.

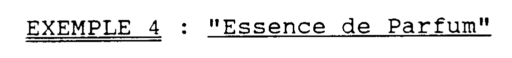

Dans un bécher, on mélange à température ambiante :
- 40 g de concentré de parfum,
- 10 à 15 g de tensioactif glycérides C8-C10 polyglycolysés,
- 12 à 20 g de cotensioactif isostéarate de polyglycérol,
- 8 à 15 g de cotensioactif DEA alcool oléique polyéthoxylé éther phosphate,
- 15 g d'huile végétale,
- 0,2 g de conservateur,
- qsp 100 g d'eau déminéralisée.

Après environ 15 minutes d'agitation à vitesse lente à température ambiante, on obtient une microémulsion stable, fluide et transparente.

Toutes ces microémulsions peuvent avoir des présentations variées, telles que microémulsions traditionnelles, mousse, aérosol, forme pressurisée..., avec une stabilité aux températures usuelles d'emploi (c'est-à-dire dans une gamme allant de - 30°C à + 80°C environ) excellente, un caractère limpide utilisable tout particulièrement dans les applications cosmétiques sur le visage, le corps ou dans les cheveux.

## Revendications

1. Microémulsion cosmétique, parfumante et non alcoolique en ce qu'elle est dépourvue d'hydrocarbure saturé avec un nombre d'atomes de carbone inférieur à 10 ou supérieur à 20 porteur d'une fonction hydroxyle en substitution d'une fonction hydrogène, cette microémulsion comprenant en mélange :
- une phase grasse ou lipophile comprenant un concentré de matières odorantes ou de parfum, la concentration en matières odorantes ou parfum étant comprise entre environ 5 et 50 % en poids du mélange,
- un agent tensio-actif à base de polyéthylène glycol,
- un premier agent cotensio-actif à base de polyglycérol,
- un deuxième agent cotensio-actif à base d'éther phosphate, et
- une phase aqueuse ou hydrophile.

2. Microémulsion selon la revendication 1, caractérisée en ce que l'agent tensio-actif est un ester de polyéthylène glycol, le polyéthylène glycol ayant un poids moléculaire moyen compris entre 100 et 1000.

3. Microémulsion selon la revendication 1 ou la revendication 2, caractérisée en ce que le premier agent cotensio-actif est un ester de polyglycérol et d'acides gras liquides, le polyglycérol ayant un poids moléculaire compris entre environ 100 et 1000.

4. Microémulsion selon l'une quelconque des revendications 1 à 3, caractérisée en ce que le deuxième agent cotensio-actif est un alkyl éther phosphate à base d'alcools gras polyéthoxylés.

5. Microémulsion selon l'une quelconque des revendications précédentes, caractérisée en ce que le deuxième agent cotensio-actif est, dans le mélange, en proportion inférieure à celle de l'agent tensio-actif.

6. Microémulsion selon l'une quelconque des revendications précédentes, caractérisée en ce que la concentration en matières odorantes est comprise entre 8 et 20 % en poids du mélange.

7. Microémulsion selon l'une quelconque des revendications précédentes, caractérisée en ce que le concentré de matières odorantes est de nature non lipidique essentiellement, tout en présentant une affinité pour les lipides.

8. Microémulsion selon l'une quelconque des revendications précédentes, caractérisée en ce que :
- l'agent tensio-actif représente entre environ 10 et 50 % en poids du mélange,
- le premier agent cotensio-actif représente entre environ 1 et 20 % en poids du mélange, et
- le deuxième agent cotensio-actif représente entre environ 0,5 et 15 % en poids du mélange.

9. Microémulsion selon l'une quelconque des revendications 1 à 7, caractérisée en ce qu'elle comprend :
- 10 à 15 % en poids du mélange de tension-actif glycérides C₈-C₁₀ polyglycolysés,
- 1 à 3 % en poids du mélange du premier cotensio-actif dioléate de polyglycérol,
- 0,5 à 2 % en poids du mélange du second cotensio-actif DEA alcool oléique polyéthoxylé éther phosphate.

10. Microémulsion selon l'une quelconque des revendications 1 à 7, caractérisée en ce qu'elle comprend :
- 20 à 26 % en poids du mélange de tensioactif glycéryl stéarate polyglycolysé,
- 2 à 6 % en poids du mélange du premier cotensio-actif dioléate de polyglycérol,
- 1 à 6 % en poids du mélange du second cotensio-actif DEA alcool oléique polyéthoxylé éther phosphate,

11. Microémulsion selon l'une quelconque des revendications 1 à 7, caractérisée en ce qu'elle comprend :
- 30 à 50 % en poids du mélange de tensioactif glycéryl stéarate polyglycolysé,
- 10 à 15 % en poids du mélange du premier cotensio-actif isostéarate de polyglycérol,
- 4 à 10 % en poids du mélange du second centensio-actif sodium dioléate polyéthoxylé éther phosphate.

12. Microémulsion selon l'une quelconque des revendications 1 à 7, caractérisée en ce qu'elle comprend :
- 10 à 15 % en poids du mélange de tensioactif glycérides C₈-C₁₀ polyglycolysés,
- 12 à 20 % en poids du mélange du premier cotensio-actif isostéarate de polyglycérol,
- 8 à 15 % en poids du mélange du second cotensio-actif DEA alcool oléique polyéthoxylé éther phosphate.

## Claims

1. Perfuming cosmetic microemulsion which is non-alcoholic in that it does not contain a saturated hydrocarbon with a number of carbon atoms below 10 or above 20 and bearing an hydroxyl function replacing a hydrogen atom, this microemulsion comprising, mixed:
- a fatty or lipophilic phase comprising a concentrate of odoriferous substances or perfume, the concentration of odoriferous substances or perfume being between approximately 5 and 50% by weight of the mixture,
- a surfactant based on polyethylene glycol,
- a first cosurfactant based on polyglycerol,
- a second cosurfactant based on ether phosphate, and
- an aqueous or hydrophilic phase.

2. Microemulsion according to Claim 1, characterized in that the surfactant is a polyethylene glycol ester, the polyethylene glycol having an average molecular weight of between 100 and 1000.

3. Microemulsion according to Claim 1 or Claim 2, characterized in that the first cosurfactant is an ester of polyglycerol and liquid fatty acids, the polyglycerol having a molecular weight of between approximately 100 and 1000.

4. Microemulsion according to any one of Claims 1 to 3, characterized in that the second cosurfactant is an alkyl ether phosphate based on polyethoxylated fatty alcohols.

5. Microemulsion according to any one of the preceding claims, characterized in that the second cosurfactant is in a smaller proportion in the mixture than that of the surfactant.

6. Microemulsion according to any one of the preceding claims, characterized in that the concentration of odoriferous substances is between 8 and 20% by weight of the mixture.

7. Microemulsion according to any one of the preceding claims, characterized in that the concentrate of odoriferous substances is essentially non-lipid in nature, while possessing an affinity for lipids.

8. Microemulsion according to any one of the preceding claims, characterized in that:
- the surfactant represents between approximately 10 and 50% by weight of the mixture,
- the first cosurfactant represents between approximately 1 and 20% by weight of the mixture, and
- the second cosurfactant represents between approximately 0.5 and 15% by weight of the mixture.

9. Microemulsion according to any one of Claims 1 to 7, characterized in that it comprises:
- 10 to 15% by weight of the mixture of surfactant polyglycol ester of C₈-C₁₀ glycerides,
- 1 to 3% by weight of the mixture of the first cosurfactant polyglyceryl dioleate,
- 0.5 to 2% by weight of the mixture of the second cosurfactant DEA polyoxyethylene oleyl ether phosphate.

10. Microemulsion according to any one of Claims 1 to 7, characterized in that it comprises:
- 20 to 26% by weight of the mixture of surfactant polyglycol ester of glyceryl stearate,
- 2 to 6% by weight of the mixture of the first cosurfactant polyglyceryl dioleate,
- 1 to 6% by weight of the mixture of the second cosurfactant DEA polyoxyethylene oleyl ether phosphate.

11. Microemulsion according to any one of Claims 1 to 7, characterized in that it comprises:
- 30 to 50% by weight of the mixture of surfactant polyglycol ester of glyceryl stearate,
- 10 to 15% by weight of the mixture of the first cosurfactant polyglyceryl isostearate,
- 4 to 10% by weight of the mixture of the second cosurfactant sodium polyoxyethylene oleyl ether phosphate.

12. Microemulsion according to any one of Claims 1 to 7, characterized in that it comprises:
- 10 to 15% by weight of the mixture of surfactant polyglycol ester of C₈-C₁₀ of glycerides,
- 12 to 20% by weight of the mixture of the first cosurfactant polyglyceryl isostearate,
- 8 to 15% by weight of the mixture of the second cosurfactant DEA polyoxyethylene oleyl ether phosphate.

## Patentansprüche

1. Kosmetische, parfümierende und nicht alkoholische Mikroemulsion ohne gesättigten Kohlenwasserstoff mit einer Anzahl von Kohlenstoffatomen, die niedriger als 10 oder höher als 20 ist, als Träger einer Hydroxylgruppe als Ersatz für eine Wasserstoffgruppe, wobei die Mikroemulsion ein Gemisch aufweist:
- eine fette oder lipophile Phase mit einem Konzentrat aus duftenden Stoffen oder aus Parfums, wobei die Konzentration an duftenden Stoffen oder Parfum ungefähr zwischen 5 und 50 Gew.-% des Gemisches angenommen wird,
- ein oberflächenaktives Mittel auf der Basis von Polyethylenglykol,
- ein erstes mitoberflächenaktives Mittel auf der Basis von Polyglycerol,
- ein zweites mitoberflächenaktives Mittel auf der Basis von Phosphatether und
- eine wäßrige oder hydrophile Phase.

2. Mikroemulsion nach Anspruch 1, dadurch gekennzeichnet, daß das oberflächenaktive Mittel ein Polyethylenglykolester ist, wobei das Polyethylenglykol ein mittleres Molekulargewicht zwischen 100 und 1000 hat.

3. Mikroemulsion nach Anspruch 1 oder Anspruch 2, dadurch gekennzeichnet, daß das erste mitoberflächenaktive Mittel ein Ester von Polyglycerol und flüssigen Fettsäuren ist, wobei das Polyglycerol ein Molekulargewicht zwischen ungefähr 100 und 1000 hat.

4. Mikroemulsion nach einem der Ansprüche 1 bis 3, dadurch gekennzeichnet, daß das zweite mitoberflächenaktive Mittel ein Alkyl-phosphatether auf der Basis von polyethoxylierten Fettalkoholen ist.

5. Mikroemulsion nach einem der vorhergehenden Ansprüche, dadurch gekennzeichnet, daß das zweite mitoberflächenaktive Mittel in dem Gemisch in einem niedrigeren Verhältnis als dem des oberflächenaktiven Mittels vorliegt.

6. Mikroemulsion nach einem der vorhergehenden Ansprüche, dadurch gekennzeichnet, daß die Konzentration an duftenden Stoffen zwischen 8 und 20 Gew.-% des Gemisches verstanden wird.

7. Mikroemulsion nach einem der vorhergehenden Ansprüche, dadurch gekennzeichnet, daß das Konzentrat aus duftenden Stoffen von im wesentlichen nicht-lipidischer Natur ist, wobei es zugleich eine Affinität für Fette aufweist.

8. Mikroemulsion nach einem der vorhergehenden Ansprüche, dadurch gekennzeichnet, daß:
- das oberflächenaktive Mittel zwischen ungefähr 10 und 50 Gew.-% des Gemisches aufweist,
- das erste mitoberflächenaktive Mittel zwischen ungefähr 1 und 20 Gew.-% des Gemisches aufweist, und
- das zweite mitoberflachenaktive Mittel zwischen ungefähr 0,5 und 15 Gew.-% des Gemisches aufweist.

9. Mikroemulsion nach einem der Ansprüche 1 bis 7, dadurch gekennzeichnet, daß sie aufweist:
- 10 bis 15 Gew.-% des Gemisches an oberflächenaktiven polyglykolysierten C₈-C₁₀-Glyceriden,
- 1 bis 3 Gew.-% des Gemisches von erstem mitoberflächenaktivem Polyglyceroldioleat,
- 0,5 bis 2 Gew.-% des Gemisches von zweitem mitoberflächenaktivem Phosphatether von polyethoxyliertem Oleylalkohol DEA,

10. Mikroemulsion nach einem der Ansprüche 1 bis 7, dadurch gekennzeichnet, daß sie aufweist:
- 20 bis 26 Gew.-% des Gemisches von oberflächenaktivem polyglykolysiertem Glycerylstearat,
- 2 bis 6 Gew.-% des Gemisches von erstem mitoberflächenaktivem Polyglyceroldioleat,
- 1 bis 6 Gew.-% des Gemisches von zweitem mitoberflächenaktivem Phosphatether von polyethoxyliertem Oleylalkohol DEA.

11. Mikroemulsion nach einem der Ansprüche 1 bis 7, dadurch gekennzeichnet, daß sie aufweist:
- 30 bis 50 Gew.-% des Gemisches an oberflächenaktivem polyglykolysiertem Glycerylstearat,
- 10 bis 15 Gew.-% des Gemisches von erstem mitoberflächenaktivem Polyglycerolisostearat,
- 4 bis 10 Gew.-% des Gemisches von zweitem mitoberflächenaktivem Phosphatether von polyethoxyliertem Natriumdioleat.

12. Mikroemulsion nach einem der Ansprüche 1 bis 7, dadurch gekennzeichnet, daß sie aufweist:
- 10 bis 15 Gew.-% des Gemisches von oberflächenaktiven polyglykolysierten C₈-C₁₀-Glyceriden,
- 12 bis 20 Gew.-% des Gemisches von erstem mitoberflächenaktivem Polyglycerolisostearat,
- 8 bis 15 Gew.-% des Gemisches von zweitem mitoberflächenaktivem Phosphatether von polyethoxyliertem Oleylalkohol DEA.
